# EUROPÄISCHE PATENTANMELDUNG

(11) **EP 3 015 452 A1**
(43) Veröffentlichungstag der Anmeldung: **04.05.2016**
(21) Anmeldenummer: 14191403.6
(22) Anmeldetag: 03.11.2014
(51) Int. Cl.: C07C 209/42, C07C 211/45, C07C 211/52, C07C 213/02, C07C 217/80, C07C 221/00, C07C 225/22, C07C 227/04, C07C 229/52, C07C 245/08

(54) **Verfahren zum Herstellen von Biphenylaminen aus Azobenzolen durch Rutheniumkatalyse**

(71) Anmelder: Bayer CropScience AG, 40789 Monheim am Rhein (DE); Georg-August-Universität Göttingen, 37073 Göttingen (DE)
(72) Erfinder: Himmler, Thomas, 51519 Odenthal (DE); Rodefeld, Lars, 51375 Leverkusen (DE); Hubrich, Jonathan, 37073 Gottingen (DE); Ackermann Lutz, 37077 Gottingen (DE)
(74) Vertreter: BIP Patents

(57) **Zusammenfassung**

Die vorliegende Erfindung betrifft ein neues Verfahren zum Herstellen von substituierten Biphenylaminen

## Beschreibung

Die vorliegende Erfindung betrifft ein neues Verfahren zum Herstellen von substituierten Biphenylaminen durch rutheniumkatalysierte Arylierung von Azobenzolen.

Biarylverbindungen, insbesondere Biphenylverbindungen, haben technische Bedeutung als Feinchemikalien, Zwischenprodukte für Pharmazeutika, optische Aufheller und Agrochemikalien.

Grundsätzliche Methoden zur Herstellung von Biarylverbindungen sowie der damit verbundenen Nachteile wurden bereits in der europäischen Patentanmeldung EP 14166058.9 offenbart und diskutiert.

Nachteilig bei diesen Verfahren sind u.a. die hohen Herstellkosten. Die übergangsmetallkatalysierten Kreuzkupplungen (z.B. nach Suzuki) erfordern relativ hohe Mengen an teuren Palladiumkatalysatoren oder aber (Bull. Korean Chem. Soc. 2000, 21, 165-166 ) die Verwendung von nahezu äquivalenten Mengen Zink, das als Abfall entsorgt werden muss. Zur Aktivierung des Zinks wird überdies das cancerogene Dibrommethan benötigt.

Es wurde auch bereits beschrieben, dass sich in ortho-Position halogenierte Azobenzole palladiumkatalysiert mit Boronsäuren in einer Suzuki-Miyaura-Reaktion arylieren lassen (siehe beispielsweise: K.Suwa et al., Tetrahedron Letters 50 (2009) 2106-8). Diese Methode hat die Nachteile der Verwendung von teuren Palladiumkatalysatoren und der Notwendigkeit der Herstellung der halogenierten Azobenzole.

Es ist ebenfalls bereits bekannt, dass sich auch nicht halogenierte Azobenzole in ortho-Position arylieren lassen (S.-I.Murahashi et al., J.Org.Chem. 43 (1978) 4099-4106; N.Taccardi et al., Eur.J.Inorg.Chem. 2007, 4645-52). Bei dieser Methode werden jedoch stöchiometrische Mengen von Palladiumkomplexen mit Azobenzolen (Herstellung siehe beispielsweise: A.C.Cope und R.W. Siekman, J.Amer.Chem.Soc. 87 (1965) 3272-3) eingesetzt, was sie unökonomisch macht.

Beschrieben wurde auch bereits, dass sich Azobenzole in Gegenwart von Rhodiumkatalysatoren mit Boronsäuren arylieren lassen (S.Miyamura et al., J.Organomet.Chem. 693 (2008) 2438-42). Rhodiumkatalysatoren sind jedoch äußerst teuer. Ferner besteht zusätzlich die Notwendigkeit der Herstellung der Boronsäuren, typischerweise aus den entsprechenden Iod- oder Bromaromaten. Letztlich sind die Ausbeuten unbefriedigend (maximal 50%) und als Produkte werden nur die ortho.ortho'-Doppelarylierungsverbindungen erhalten.

Die Aufgabe der vorliegenden Erfindung bestand also in der Bereitstellung eines neuen Verfahrens, durch das Biphenylamine mit hoher Gesamtausbeute und hoher Reinheit ohne die Verwendung von teuren Palladium- oder Rhodiumkatalysatoren und unter technisch bevorzugten Reaktionsbedingungen erhalten werden können.

Gegenstand der vorliegenden Erfindung ist demnach ein Verfahren zum Herstellen von Biphenylaminen der allgemeinen Formel (I) 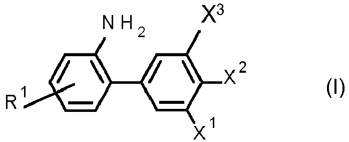 in welcher
- R¹: für Wasserstoff, Hydroxy, Fluor, Chlor, C₁-C₄-Alkyl, C₁-C₄-Alkoxy, C₁-C₄-Alkylthio oder C₁-C₄-Halogenalkyl steht,
- X¹: für Wasserstoff" Alkoxy, Alkanoyl, Alkylcarboxylat, Fluor oder Chlor steht,
- X²: für Wasserstoff, Alkoxy, Alkanoyl, Alkylcarboxylat, Fluor oder Chlor steht.,
- X³: für Wasserstoff, Alkoxy, Alkanoyl, Alkylcarboxylat, Fluor oder Chlor steht.
dadurch gekennzeichnet, dass man
(1) in einem ersten Schritt
   Azobenzole der Formel (II) 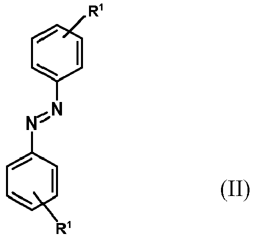 in welcher
   - R¹: die oben angegebene Bedeutung hat,
   mit einer aromatischen Verbindung der Formel (III) 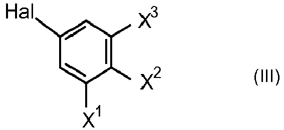 in welcher
   X¹, X² und X³ die oben angegebenen Bedeutungen haben,
   und
   Hal für Iod, Brom oder Chlor
   steht,
   in Gegenwart eines Katalysatorsystems bestehend aus einem Rutheniumkatalysator, einem Aktivierungsmittel und einer Base umsetzt,
   und (2) in einer zweiten Stufe die so erhaltenen Azobenzole der Formel (IV) 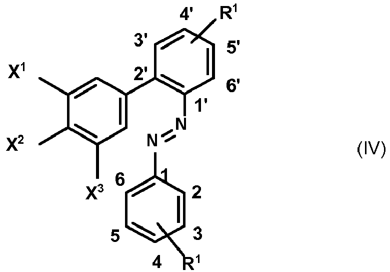 in welcher R¹, X¹, X² und X³ die oben angegebenen Bedeutungen haben
   und die Ziffern 1 bis 6 bzw. 1' bis 6' die Positionen des Restes R¹ in den in der Tabelle 1 angegebenen Verbindungen sowie bei Verweis auf die Position in Formel IV in der Beschreibung definieren,
zu den Biphenylaminen der Formel (I) hydriert.

C₁-C₄-Alkyl umfaßt Methyl, Ethyl, Propyl und Isopropyl, Butyl, Isobutyl und tert.-Butyl und ist besonders bevorzugt Methyl.

C₁-C₄-Alkoxy umfaßt Methoxy, Ethoxy, Propoxy, Isopropoxy und Butoxy und ist besonders bevorzugt Methoxy.

Überraschenderweise lassen sich durch diese Reaktionssequenz die Biphenylamine der Formel (I) in guten Ausbeuten ohne die Verwendung von halogenierten Azobenzolen, ohne Verwendung teurer Palladium- oder Rhodiumkatalysatoren, ohne die Notwendigkeit der Herstellung von Boronsäuren, und unter technisch vorteilhaften Reaktionsbedingungen herstellen.

Verwendet man Azobenzol und Brombenzol als Ausgangsstoffe, so kann das erfindungsgemäße Verfahren durch das folgende Formelschema beispielhaft veranschaulicht werden.

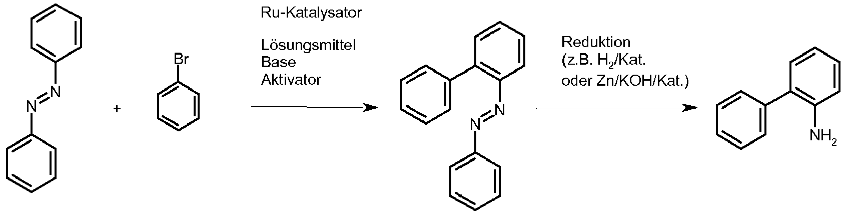

Bevorzugt ist die Durchführung des erfindungsgemäßen Verfahrens unter Verwendung von Ausgangsstoffen, in denen die angegeben Reste jeweils folgende Bedeutungen haben. Die bevorzugten, besonders bevorzugten und ganz besonders bevorzugten Bedeutungen gelten für alle Verbindungen, in denen die jeweiligen Reste vorkommen:
- R¹: steht bevorzugt Wasserstoff, Fluor, Chlor, C₁-C₄-Alkyl, C₁-C₄-Alkoxy.
- R¹: steht weiter bevorzugt für Fluor, C₁-C₄-Alkyl und C₁-C₄-Alkoxy, wobei der Substituent bevorzugt in in 3'-, 4'- oder 5'- Position, weiter bevorzugt in 4'- oder 5'-Position und besonders bevorzugt in 5'- Position steht [vgl. z.B. Formel (IV)].
- R¹: steht besonders bevorzugt für C₁-C₄-Alkyl und C₁-C₄-Alkoxy, wobei der Substituent in 4'-oder 5'-Position und besonders bevorzugt in 5'- Position steht [vgl. z.B. Formel (IV)].

In den vorangehenden Definitionen für R¹ ist C₁-C₄-Alkyl bevorzugt ausgewählt aus der Gruppe, die Methyl, Ethyl und Isopropyl umfaßt, und C₁-C₄-Alkoxy bevorzugt ausgewählt aus der Gruppe, die Methoxy und Ethoxy umfaßt.

In einer alternativen Ausführungsform steht
- R¹: bevorzugt für Trifluormethyl, wobei Trifluormethyl bevorzugt in 4'- oder 5'-Position, weiter bevorzugt in 5'- Position der jeweiligen Verbindung steht.

In einer weiteren alternativen Ausführungsform steht
- R¹: bevorzugt für Methoxy oder Methylthio, bevorzugt in 4'-, 5'- oder 6'-Position, weiter bevorzugt in 5'- Position der jeweiligen Verbindung stehen.
- X¹: steht bevorzugt für Alkoxy, Alkanoyl, Alkylcarboxylat, oder Chlor.
- X¹: steht besonders bevorzugt für Alkoxy, Alkanoyl, Alkylcarboxylat und ganz besonders bevorzugt für Alkylcarboxylat.
- X²: steht bevorzugt für Alkoxy, Alkanoyl, Alkylcarboxylat, oder Chlor.
- X²: steht besonders bevorzugt für Alkoxy, Alkanoyl, Alkylcarboxylat und ganz besonders bevorzugt für Alkylcarboxylat.
- X³: steht bevorzugt für Alkoxy, Alkanoyl, Alkylcarboxylat, oder Chlor.
- X³: steht besonders bevorzugt für Alkoxy, Alkanoyl, Alkylcarboxylat und ganz besonders bevorzugt für Alkylcarboxylat.

Alkylcarboxylat in den vorstehenden Definitionen ist ganz besonders bevorzugt ausgewählt aus der Gruppe, die Methyl-, Ethyl- und Isopropyl-Carboxylat umfaßt. Alkanoyl in den vorstehenden Definitionen ist ganz besonders bevorzugt ausgewählt aus der Gruppe, die -COMe, -COEt, -COiPr, -COPr, -COButyl, -COisobutyl, -COtert.-butyl umfaßt, wobei Me, Et, und Pr die üblichen Bedeutungen Methyl, Ethyl und Propyl haben.

Die bei der Durchführung des erfindungsgemäßen Verfahrens in der ersten Stufe als Ausgangsstoffe zu verwendenden Azobenzole der Formel (II) sind bekannt oder können nach bekannten Verfahren erhalten werden.

Die erste Stufe des erfindungsgemäßen Verfahrens wird in Gegenwart eines Rutheniumkatalysators durchgeführt. Als Rutheniumkatalysator werden beispielsweise Rutheniumkomplexe wie [{RuC₂(p-Cymol)}₂], [{RuCl₂(Cumol)}₂] [{RuCl₂(Benzol)}₂], [{RuCl₂(C₆Me₆)}₂], [Cp*Ru(PPh₃)₂Cl] (Cp*=Pentamethylcyclopentadienyl eingesetzt. Bevorzugt verwendet man [{RuCl₂(p-Cymol)}₂].

Die Menge an Rutheniumkatalysator kann in weiten Grenzen variiert werden. Üblicherweise setzt man Mengen von 0,1 bis 30 Molprozent, bezogen auf die aromatische Verbindung der Formel (III), des entsprechenden Komplexes ein. Bevorzugt setzt man 1 bis 20 Molprozent des entsprechenden Komplexes ein, weiter bevorzugt 1 bis 10 Molprozent. Bei der Durchführung der ersten Stufe des erfindungsgemäßen Verfahrens wird in Gegenwart eines Aktivierungsmittels gearbeitet.
Das Aktivierungsmittel ist vorzugsweise eine Säure, weiter bevorzugt eine Carbonsäure. Beispielhaft seien genannt Carbonsäuren wie Ameisensäure, Essigsäure, Propionsäure, Pivalsäure, Benzoesäure, 2-Methylbenzoesäure, 3-Methylbenzoesäure, 4-Methylbenzoesäure, 2,3-Dimethylbenzoesäure, 2,4-Dimethylbenzoesäure, 2,5-Dimethylbenzoesäure, 2,6-Dimethylbenzoesäure, 3,4-Dimethylbenzoesäure, 3,5-Dimethylbenzoesäure, 2,4,6-Trimethylbenzoesäure, 2,3,4-Trimethylbenzoesäure, 3,4,5-Trimethylbenzoesäure, 2,3,5-Trimethylbenzoesäure, 2,3,6-Trimethylbenzoesäure, Phenylessigsäure, 2-Methylphenylessigsäure. 3-Methylphenylessigsäure, 4-Methylphenylessigsäure, 2,5-Dimethylphenylessigsäure, 2,3,6-Trimethylphenylessigsäure, 2,3,5,6-Tetramethylphenylessigsäure, 2,3,4,6-Tetramethylphenylessigsäure, 2-Chlorphenylessigsäure, 3-Chlorphenylessigsäure, 4-Chlorphenylessigsäure, 2,4-Dichlorphenylessigsäure.
Bevorzugt verwendet man 2,4,6-Trimethylbenzoesäure (MesCO₂H).

Das Aktivierungsmittel wird in Mengen von 0,1 bis 100 Molprozent, bezogen auf die aromatische Verbindung der Formel (III), eingesetzt. Bevorzugt werden 1 bis 50 Molprozent, weiter bevorzugt 10 bis 40 Molprozent eingesetzt.

Bei der Durchführung der ersten Stufe des erfindungsgemäßen Verfahrens arbeitet man in Gegenwart einer Base. Als Basen kommen organische oder anorganischen Basen in Betracht. Beispielhaft seien genannt Ammoniak, Trimethylamin, Triethylamin, Tributylamin, Di-isopropylethylamin, Pyrrolidin, Piperidin, Morpholin, Pyridin, 2-Picolin, 3-Picolin, 4-Picolin, 2,3-Lutidin, 2,4-Lutidin, 2,5-Lutidin, 2,6-Lutidin, 3,4-Lutidin, 3,5-Lutidin, 5-Ethyl-2-methyl-pyridin, Chinolin, Lithiumhydroxid, Natriumhydroxid, Kaliumhydroxid, Lithiumcarbonat, Natriumcarbonat, Kaliumcarbonat, Lithiumhydrogencarbonat, Natriumhydrogencarbonat, Kaliumhydrogencarbonat, Calciumcarbonat, Magnesiumcarbonat, Lithiumacetat, Natriumacetat, Kaliumacetat, Lithiumpivalat, Natriumpivalat, Kaliumpivalat. Bevorzugt verwendet man anorganische Basen; besonders bevorzugt Natriumcarbonat, Kaliumcarbonat, Natriumhydrogencarbonat, Kaliumhydrogencarbonat, Calciumcarbonat, Magnesiumcarbonat, Natriumacetat, Kaliumacetat, Natriumpivalat und Kaliumpivalat.
Besonders bevorzugt wird Kaliumcarbonat verwendet.

Bei der Durchführung der ersten Stufe des erfindungsgemäßen Verfahrens arbeitet man in Lösungsmitteln oder Lösungsmittelgemischen. Beispielhaft genannt seien:
Ketone wie Aceton, Diethylketon, Methyl-ethyl-keton und Methyl-isobutyl-keton;
Nitrile wie z.B. Acetonitril und Butyronitril;
Ether wie z.B. Dimethoxyethan (DME), Tetrahydrofuran (THF), 2-Methyl-THF und 1,4-Dioxan; ve Alkohole wie z.B. n-Propanol, Isopropanol, Tertiärbutanol, Isoamylalkohol oder Tertiäramylalkohol; Kohlenwasserstoffe und halogenierte Kohlenwasserstoffe wie Hexan, Heptan, Cyclohexan, Methylcyclohexan, Toluol, ortho-Xylol, meta-Xylol, para-Xylol, Mesitylen, Chlorbenzol, ortho-Dichlorbenzol oder Nitrobenzol.

Bevorzugt ist das Lösungsmittel ausgewählt aus der Gruppe, die Ether, aromatische Kohlenwasserstoffe, chlorierte aromatische Kohlenwasserstoffe und verzweigte Alkohole, oder Gemische dieser Lösungsmittel umfaßt.

Besonders bevorzugt ist das Lösungsmittel ausgewählt aus der Gruppe, die 1,4-Dioxan, THF, 2-Me-THF, DME, Toluol, ortho-Xylol, meta-Xylol, para-Xylol, Mesitylen oder Tertiäramylalkohol, oder Gemische dieser Lösungsmittel.
Ganz besonders bevorzugt sind die Lösungsmittel 1,4-Dioxan, Toluol, ortho-Xylol, meta-Xylol, para-Xylol oder Gemische dieser Lösungsmittel.
Als Lösungsmittel ungeeignet erwiesen haben sich Methanol, N,N-Dialkylalkanamide, wie z.B. N-Methylpyrrolidon, Lactone, wie z.B. γ-Valerolacton, Wasser, Dimethylsulfoxid (DMSO) und Carbonsäuren wie z.B. Essigsäure.

Bei der Durchführung der ersten Stufe des erfindungsgemäßen Verfahrens arbeitet man im Allgemeinen bei Temperaturen im Bereich von 20°C bis 220°C, vorzugsweise im Bereich von 50°C bis 180°C, weiter bevorzugt im Bereich von 80°C bis 150°C.

Bei der Durchführung der ersten Stufe des erfindungsgemäßen Verfahrens setzt man auf 1 Mol an Azobenzol der Formel (II) im Allgemeinen einen Unterschuss bis zu äquimolaren Mengen an Halogenaromaten der Formel (III) ein. Das Molverhältnis von Azobenzol der Formel (II) zu Halogenaromat der Formel (III) ist im Allgemeinen 1 zu 0,4 bis 1, bevorzugt 1 zu 0,45 bis 0,9.

Die erste Stufe des erfindungsgemäßen Verfahrens wird, wenn nicht anders angegeben, im Allgemeinen unter Normaldruck durchgeführt. Es ist jedoch auch möglich, unter erhöhtem oder unter vermindertem Druck zu arbeiten. Bevorzugt wird unter Normaldruck gearbeitet.

Die zweite Stufe des erfindungsgemäßen Verfahrens, d.h. die Reduktion des Azobenzols der Formel (II) zu einem Biphenylamin der Formel (I) kann nach prinzipiell bekannten Verfahren durchgeführt werden, beispielsweise mittels Zink und Ammoniumformiat (S.Gowda et al., Tetrahedron Letters 43 (2002) 1329-31); Eisen und Calciumchlorid (S.Chandrappa et al., Synlett 2010, 3019-22); rutheniumkatalysierte Transferhydrierung (M.Beller et al., Chem.Eur.J. 2011, 17, 14375-79); rutheniumkatalysierte Reduktion mit Zink und KOH (T.Schabel et al., Org.Lett. 15 (2013) 2858-61):

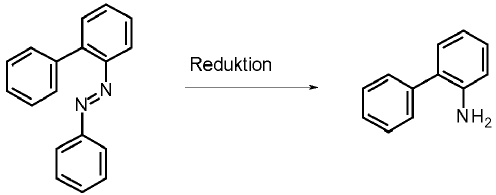

Bevorzugt benutzt man rutheniumkatalysierte Verfahren.

Besonders bevorzugt führt man die Reduktion in Gegenwart des Rutheniumkatalysators durch, der bereits für die erste Stufe des erfindungsgemäßen Verfahrens verwendet wurde.

Ganz besonders bevorzugt führt man die zweite Stufe des erfindungsgemäßen Verfahrens dabei dergestalt durch, dass sie direkt nach der ersten Stufe des erfindungsgemäßen Verfahrens ohne Isolierung des Azobenzols der Formel (II) in einer Eintopfreaktion erfolgt.

In einer besonders bevorzugten Ausführungsform ist das Lösungsmittel ausgewählt aus der Gruppe, die 1,4-Dioxan, Toluol, ortho-Xylol, meta-Xylol, para-Xylol oder Gemische dieser Lösungsmittel umfaßt und der Aktivator 2,4,6-Trimethlylbenzoesäure, die aromatische Verbindung der Formel (III) eine bromierte aromatische Verbindung, die Base Kaliumcarbonat und der Katalysator [{RuCl₂(p-Cymol)}₂] ist.

Die Biphenylamine der Formel (I) sind wertvolle Zwischenprodukte zur Herstellung fungizider Wirkstoffe (vgl. WO 03/070705).

Bevorzugte Ausführungsformen von Verbindungen der Formel (IV) im Sinne der vorliegenden Erfindung sind (die Zahlen zu R¹ geben jeweils die Position an und HAL das Halogen der Ausgangsverbindung III):

**Tabelle 1**

| | R¹ | X¹ | X² | X³ | HAL |
|---|---|---|---|---|---|
| V1 | Me(3/5') | H | CO₂Me | H | Br |
| V8 | Me(3/5') | H | COMe | H | Br |
| V9 | OMe(3/5') | H | COMe | H | Br |
| V10 | Me(2/6') | H | COMe | H | Br |
| V11 | Me(3/5') | H | CO₂Et | H | Br |
| V12 | Me(3/5') | H | OMe | H | Br |
| V13 | H | H | H | H | Br |
| V14 | H | H | CO₂Me | H | Br |
| V15 | Me(2/6') | H | CO₂Me | H | Br |
| V16 | Me(4/4') | H | CO₂Me | H | Br |
| V17 | Et(3/5') | H | CO₂Me | H | Br |
| V18 | iPr(3/5') | H | CO₂Me | H | Br |
| V19 | OMe(3/5') | H | CO₂Me | H | Br |
| V20 | H | Cl | Cl | H | Br |
| V21 | Me(3/5') | H | Cl | H | Br |
| V22 | Me(3/5') | Cl | Cl | H | Br |
| V23 | Et(3/5') | H | COMe | H | Br |
| V24 | iPr(3/5') | H | COMe | H | Br |
| V25 | Et(3/5') | H | OMe | H | Br |
| V26 | iPr(3/5') | H | OMe | H | Br |
| V27 | Me(3/5') | H | OEt | H | Br |
| V28 | Me(3/5') | H | OEt | H | Br |
| V29 | Me(3/5') | H | OEt | H | Br |
| V30 | Me(3/5') | H | OiPr | H | Br |
| V31 | Me(3/5') | H | OiPr | H | Br |
| V32 | Me(3/5') | H | OiPr | H | Br |
| V33 | Me(3/5') | H | CO₂Et | H | Br |
| V34 | Me(3/5') | H | CO₂iPr | H | Br |
| V35 | OMe(3/5') | H | CO₂Et | H | Br |
| V36 | OMe(3/5') | H | CO₂iPr | H | Br |
| V37 | OMe(3/5') | H | Cl | H | Br |
| V38 | OMe(3/5') | Cl | Cl | H | Br |
| V39 | H | Cl | Cl | Cl | I |
| V40 | H | Cl | H | H | I |
| V41 | H | H | Cl | H | I |
| V42 | H | H | F | H | Br |
| V43 | H | F | F | H | Br |
| V44 | H | F | F | F | Br |
| V45 | H | F | F | F | I |
| V46 | Me(4/4') | H | Cl | H | Br |
| V47 | Me(4/4') | Cl | Cl | H | Br |
| V48 | Me(4/4') | Cl | Cl | Cl | Br |
| V49 | Me(4/4') | H | F | H | Br |
| V50 | Me(4/4') | F | F | H | Br |
| V51 | Me(4/4') | F | F | F | Br |
| V52 | OMe(4/4') | H | Cl | H | Br |
| V53 | OMe(4/4') | Cl | Cl | H | Br |
| V54 | OMe(4/4') | Cl | Cl | Cl | Br |
| V55 | OMe(4/4') | H | F | H | Br |
| V56 | OMe(4/4') | F | F | H | Br |
| V57 | OMe(4/4') | F | F | F | Br |
| V58 | Et(4/4') | Cl | Cl | Cl | Br |
| V59 | OEt(4/4') | Cl | Cl | H | Br |
| V60 | OEt(4/4') | F | F | F | Br |

### Herstellungsbeispiele

### Beispiel 1: Methyl-4'-methyl-2'-[(E)-(3-methylphenyl)diazenyl]biphenyl-4-carboxylat

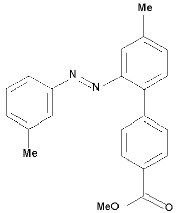

In einem ausgeheizten Reaktionsgefäß wurde eine Suspension bestehend aus (E)-Bis(3-methylphenyl)diazen (210 mg, 1.0 mmol), [{RuCl₂(p-Cymol)}₂] (15.3 mg, 5.0 mol %), MesCO₂H (24.6 mg, 30 mol %), K₂CO₃ (138 mg, 1.0 mmol) und Methyl-4-brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei Raumtemperatur mit Dichlormethan (DCM) (75 mL) verdünnt, über Celite und Kieselgel filtriert und das Filtrat eingeengt. Das so erhaltene Rohprodukt wurde durch Chromatografie an Kieselgel gereinigt (n-Hexan/DCM: 7/3). Es wurden 150 mg Methyl-4'-methyl-2'-[(E)-(3-methylphenyl)diazenyl]biphenyl-4-carboxylat als oranger Feststoff erhalten (87% der Theorie).
Smp. = 136-137 °C. ¹H-NMR (CDCl₃, 300 MHz): δ = 8.10 (d, J = 8.0 Hz, 2H), 7.64 (s, 1H), 7.60-7.53 (m, 4H), 7.49 (d, J = 7.8 Hz, 1H), 7.40-7.32 (m, 2H), 7.30-7.24 (m, 1H), 3.96 (s, 3H), 2.48 (s, 3H), 2.42 (s, 3H). ¹³C-NMR (CDCl₃, 126 MHz): δ = 167.0 (Cq), 152.7 (Cq), 149.3 (Cq), 143.6 (Cq), 138.8 (Cq), 138.7 (Cq), 137.1 (Cq), 131.7 (CH), 131.5 (CH), 130.7 (CH), 130.4 (CH), 128.8 (CH), 128.7 (CH), 128.5 (Cq), 124.2 (CH), 119.8 (CH), 116.1 (CH), 52.1 (CH₃), 21.4 (CH₃), 21.3 (CH₃). IR (neat): 3030, 2951, 2914, 2850, 1721, 1606, 1438, 1279, 1106, 829, 790, 704, 533, 475, 436 cm-1. MS (EI) m/z (relative Intensität): 344 ([M+] 60), 329 (80), 285 (38), 225 (43), 165 (87), 91 (100), 65 (25), 43 (22). HR-MS (EI) m/z berechnet für C₂₂H₂₀N₂O₂ [M+] 344.1525, gefunden 344.1511.

### Beispiel 2: Methyl-4'-methyl-2'-[(E)-(3-methylphenyl)diazenyl]biphenyl-4-carboxylat

Der Versuch wurde wie für Beispiel 1 beschrieben durchgeführt, mit dem Unterschied, dass Toluol anstelle von 1,4-Dioxan als Lösungsmittel verwendet wurde. Die Ausbeute betrug 83% der Theorie.

### Beispiel 3: Methyl-4'-methyl-2'-[(E)-(3-methylphenyl)diazenyl]biphenyl-4-carboxylat

Der Versuch wurde wie für Beispiel 1 beschrieben durchgeführt, mit dem Unterschied, dass ortho-Xylol anstelle von 1,4-Dioxan als Lösungsmittel verwendet wurde. Die Ausbeute betrug 84% der Theorie.

### Beispiel 4: Methyl-4'-methyl-2'-[(E)-(3-methylphenyl)diazenyl]biphenyl-4-carboxylat

Der Versuch wurde wie für Beispiel 1 beschrieben durchgeführt, mit dem Unterschied, dass Tertiäramylalkohol anstelle von 1,4-Dioxan als Lösungsmittel verwendet wurde. Die Ausbeute betrug 75% der Theorie.

### Beispiel 5: Methyl-4'-methyl-2'-[(E)-(3-methylphenyl)diazenyl]biphenyl-4-carboxylat

Der Versuch wurde wie für Beispiel 1 beschrieben durchgeführt, mit dem Unterschied, dass Natriumcarbonat anstelle von Kaliumcarbonat als Base verwendet wurde. Die Ausbeute betrug 75% der Theorie.

### Beispiel 6: Methyl-4'-methyl-2'-[(E)-(3-methylphenyl)diazenyl]biphenyl-4-carboxylat

Der Versuch wurde wie für Beispiel 1 beschrieben durchgeführt, mit dem Unterschied, dass Kaliumacetat anstelle von MesCO₂H als Additiv verwendet wurde. Die Ausbeute betrug 79% der Theorie.

### Beispiel 7: Methyl-4'-methyl-2'-[(E)-(3-methylphenyl)diazenyl]biphenyl-4-carboxylat

Der Versuch wurde wie für Beispiel 1 beschrieben durchgeführt, mit dem Unterschied, dass Pivalsäure anstelle von MesCO₂H als Additiv verwendet wurde. Die Ausbeute betrug 76% der Theorie.

### Beispiel 8: 1-{4'-Methyl-2'-[(E)-(3-methylphenyl)diazenyl]biphenyl-4-yl}ethanon

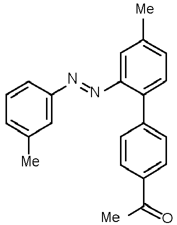

In einem ausgeheizten Reaktionsgefäß wurde eine Suspension bestehend aus (E)-1,2-Di-m-tolyldiazen (210 mg, 1.0 mmol), [{RuCl₂(p-Cymol)}₂] (15.3 mg, 5.0 mol %), MesCO₂H (24.6 mg, 30 mol %), K₂CO₃ (138 mg, 1.0 mmol) und 4-Bromacetophenon (99.5 mg, 0.5 mmol) in trockenem 1,4-Dioxan (2.0 mL) bei 120 °C für 18 h in einer Stickstoffatmosphäre gerührt. Das Reaktionsgemisch wurde dann bei Raumtemperatur mit Dichlormethan (DCM) (75 mL) verdünnt, über Celite und Kieselgel filtriert und das Filtrat eingeengt. Das so erhaltene Rohprodukt wurde durch Chromatografie an Kieselgel gereinigt (n-Hexan/DCM: 7/3). Es wurden 106 mg 1-{4'-Methyl-2'-[(E)-(3-methylphenyl)diazenyl]biphenyl-4-yl}ethanon erhalten (65% der Theorie).
Smp. = 123-124 °C. ¹H-NMR (CDCl₃, 300 MHz): δ = 8.00 (d, *J=* 8.6 Hz, 2H), 7.64-7.50 (m, 5H), 7.46 (d, *J=* 7.8 Hz, 1H), 7.39-7.29 (m, 2H), 7.28-7.21 (m, 1H), 2.64 (s, 3H), 2.47 (s, 3H), 2.40 (s, 3H). ¹³C-NMR (CDCl₃, 126 MHz): δ = 198.1 (Cq), 152.9 (Cq), 149.6 (Cq), 144.0 (Cq), 139.0 (Cq), 138.9 (Cq), 137.2 (Cq), 135.6 (Cq), 131.9 (CH), 131.7 (CH), 131.0 (CH), 130.5 (CH), 129.0 (CH), 127.6 (CH), 124.4 (CH), 119.9 (CH), 116.3 (CH), 26.5 (CH₃), 21.2 (CH₃), 21.1 (CH₃). IR (neat): 2914, 2856, 2723, 1679, 1600, 1266, 819, 797, 688, 599 cm⁻¹. MS (EI) *m*/*z* (relative Intensität): 328 ([M⁺] 100), 285 (44), 209 (25), 165 (41), 91 (83), 65 (19), 65 (20), 43 (76). HR-MS (EI) *m*/*z* berechnet für C₂₂H₂₀N₂O [M⁺] 328.1576, gefunden 328.1572.

### Beispiel 9: 1-{4'-Methoxy-2'-[(E)-(3-methoxyphenyl)diazenyl]biphenyl-4-yl}ethanon

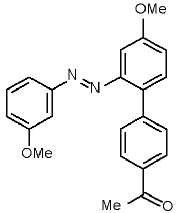

In einem ausgeheizten Reaktionsgefäß wurde eine Suspension bestehend aus ((E)-Bis(3-methoxyphenyl)diazen (242 mg, 1.0 mmol), [{RuCl₂(p-Cymol)}₂] (15.3 mg, 5.0 mol %), MesCO₂H (24.6 mg, 30 mol %), K₂CO₃ (138 mg, 1.0 mmol) und 4-Bromacetophenon (99.5 mg, 0.5 mmol) in trockenem 1,4-Dioxan (2.0 mL) bei 120 °C für 18 h in einer Stickstoffatmosphäre gerührt. Das Reaktionsgemisch wurde dann bei Raumtemperatur mit Dichlormethan (DCM) (75 mL) verdünnt, über Celite und Kieselgel filtriert und das Filtrat eingeengt. Das so erhaltene Rohprodukt wurde durch Chromatografie an Kieselgel gereinigt (n-Hexan/DCM: 7/3). Es wurden 96 mg 1-{4'-Methoxy-2'-[(E)-(3-methoxyphenyl)diazenyl]biphenyl-4-yl}ethanon erhalten (53% der Theorie).
Smp. = 155-156 °C. ¹H-NMR (CDCl₃, 300 MHz): δ = 7.98 (d, *J =* 8.5 Hz, 2H), 7.61-7.22 (m, 7H), 7.13 (dd, *J=* 8.6, 2.7 Hz, 1H), 7.00 (ddd, *J*= 8.0, 2.7, 1.2 Hz, 1H), 3.91 (s, 3H), 3.78 (s, 3H), 2.63 (s, 3H). ¹³C-NMR (CDCl₃, 126 MHz): δ = 198.1 (Cq), 160.4 (Cq), 160.2 (Cq), 154.0 (Cq), 150.2 (Cq), 143.8 (Cq), 135.4 (Cq), 133.4 (Cq), 131.6 (CH), 131.1 (CH), 129.9 (CH), 127.6 (CH), 118.5 (CH), 118.0 (CH), 117.4 (CH), 106.4 (CH), 99.4 (CH), 55.6 (CH₃), 55.3 (CH₃), 26.6 (CH₃). IR (neat): 3068, 3005, 2961, 2940, 2915, 2834, 1673, 1604, 1513, 1269, 1132, 1040, 887, 819, 782, 634cm⁻¹. MS (EI) *m*/*z* (relative Intensität): 360 ([M⁺] 100), 317 (57), 139 (38), 107 (53), 92 (24), 77 (30), 43 (54). HR-MS (EI) *m*/*z* berechnet für C₂₂H₂₀N₂O₃ [M⁺] 360.1474, gefunden 360.1466.

### Beispiel 10: 1-{3'-Methyl-2'-[(E)-(2-methylphenyl)diazenyl]biphenyl-4-yl}ethanon

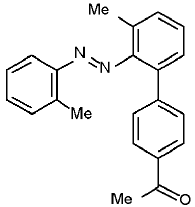

In einem ausgeheizten Reaktionsgefäß wurde eine Suspension bestehend aus (E)-Bis(2-methylphenyl)diazen (210 mg, 1.0 mmol), [{RuCl₂(p-Cymol)}₂] (15.3 mg, 5.0 mol %), MesCO₂H (24.6 mg, 30 mol %), K₂CO₃ (138 mg, 1.0 mmol) und 4-Bromacetophenon (99.5 mg, 0.5 mmol) in trockenem 1,4-Dioxan (2.0 mL) bei 120 °C für 18 h in einer Stickstoffatmosphäre gerührt. Das Reaktionsgemisch wurde dann bei Raumtemperatur mit Dichlormethan (DCM) (75 mL) verdünnt, über Celite und Kieselgel filtriert und das Filtrat eingeengt. Das so erhaltene Rohprodukt wurde durch Chromatografie an Kieselgel gereinigt (n-Hexan/DCM: 7/3). Es wurden 87 mg 1-{3'-Methyl-2'-[(E)-(2-methylphenyl)diazenyl]biphenyl-4-yl}ethanon erhalten (53% der Theorie).
Smp. = 125-126 °C. ¹H-NMR (CDCl₃, 300 MHz): δ = 7.89 (d, *J=* 8.6 Hz, 2H), 7.38-7.20 (m, 9H), 2.59 (s, 3H), 2.46 (s, 3H), 2.27 (s, 3H). ¹³C-NMR (CDCl₃, 126 MHz): δ = 198.0 (Cq), 151.0 (Cq), 150.8 (Cq), 145.7 (Cq), 138.5 (Cq), 135.2 (Cq), 134.7 (Cq), 131.5 (CH), 131.4 (CH), 131.3 (CH), 130.9 (Cq), 130.4 (CH), 128.9 (CH), 128.2 (CH), 128.0 (CH), 126.4 (CH), 115.0 (CH), 26.5 (CH₃), 19.1 (CH₃), 17.0 (CH₃). IR (neat): 3054, 2961, 2923, 1679, 1603, 1356, 1264, 955, 766, 600 cm⁻¹. MS (EI) *m*/*z* (relative Intensität): 328 ([M⁺] 100), 285 (32), 209 (25), 165 (45), 91 (98), 65 (27), 43 (90). HR-MS (EI) *m*/*z* berechnet für C₂₂H₂₀N₂O [M⁺] 328.1576, gefunden 328.1569.

### Beispiel 11: Ethyl-4'-methyl-2'-[(E)-(3-methylphenyl)diazenyl]biphenyl-4-carboxylat

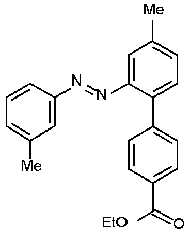

In einem ausgeheizten Reaktionsgefäß wurde eine Suspension bestehend aus (E)-Bis(3-methylphenyl)diazen (210 mg, 1.0 mmol), [{RuCl₂(p-Cymol)}₂] (15.3 mg, 5.0 mol %), MesCO₂H (24.6 mg, 30 mol %), K₂CO₃ (138 mg, 1.0 mmol) und Ethyl-4-brombenzoat (107 mg, 0.5 mmol) in trockenem 1,4-Dioxan (2.0 mL) bei 120 °C für 18 h in einer Stickstoffatmosphäre gerührt. Das Reaktionsgemisch wurde dann bei Raumtemperatur mit Dichlormethan (DCM) (75 mL) verdünnt, über Celite und Kieselgel filtriert und das Filtrat eingeengt. Das so erhaltene Rohprodukt wurde durch Chromatografie an Kieselgel gereinigt (n-Hexan/DCM: 7/3). Es wurden 113 mg Ethyl-4'-methyl-2'-[(E)-(3-methylphenyl)diazenyl]biphenyl-4-carboxylat erhalten (63% der Theorie).
Smp. = 94-95 °C. ¹H-NMR (CDCl₃, 300 MHz): δ = 8.09 (d, *J=* 8.5 Hz, 2H), 7.63-7.60 (m, 1H), 7.58-7.50 (m, 4H), 7.47 (d, *J* = 7.9 Hz, 1H), 7.39-7.30 (m, 2H), 7.28-7.22 (m, 1H), 4.41 (q, *J= 7.1* Hz, 2H), 2.47 (s, 3H), 2.41 (s, 3H), 1.41 (t, *J* = 7.1 Hz, 3H). ¹³C-NMR (CDCl₃, 126 MHz): δ=166.8 (Cq), 153.0 (Cq), 149.6 (Cq), 143.7 (Cq), 139.0 (Cq), 138.9 (Cq), 137.4 (Cq), 131.9 (CH), 131.7 (CH), 130.8 (CH), 130.6 (CH), 129.0 (Cq), 128.9 (CH), 128.8 (CH), 124.4 (CH), 119.9 (CH), 116.3 (CH), 60.9 (CH₂), 21.3 (CH₃), 21.1 (CH₃), 14.3 (CH₃). IR (neat): 2979, 2921, 2867, 1713, 1607, 1268, 1180, 1100, 775, 688 cm⁻¹. MS (EI) *m*/*z* (relative Intensität): 358 ([M⁺] 47), 329 (100), 285 (37), 239 (19), 211 (17), 165 (60), 91 (80), 65 (14). HR-MS (EI) *m*/*z* berechnet für C₂₃H₂₂N₂O₂ [M⁺] 358.1681, gefunden 358.1669.

### Beispiel 12: (E)-1-(4'-Methoxy-4-methylbiphenyl-2-yl)-2-(3-methylphenyl)diazen

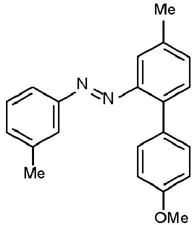

In einem ausgeheizten Reaktionsgefäß wurde eine Suspension bestehend aus (E)-Bis(3-methylphenyl)diazen (210 mg, 1.0 mmol), [{RuCl₂(p-Cymol)}₂] (15.3 mg, 5.0 mol %), MesCO₂H (24.6 mg, 30 mol %), K₂CO₃ (138 mg, 1.0 mmol) und 4-Bromanisol (93.5 mg, 0.5 mmol) in trockenem 1,4-Dioxan (2.0 mL) bei 120 °C für 18 h in einer Stickstoffatmosphäre gerührt. Das Reaktionsgemisch wurde dann bei Raumtemperatur mit Dichlormethan (DCM) (75 mL) verdünnt, über Celite und Kieselgel filtriert und das Filtrat eingeengt. Das so erhaltene Rohprodukt wurde durch Chromatografie an Kieselgel gereinigt (n-Hexan/DCM: 7/3). Es wurden 106 mg (E)-1-(4'-Methoxy-4-methylbiphenyl-2-yl)-2-(3-methylphenyl)diazen erhalten (67% der Theorie).
Smp. = 121-122 °C. ¹H-NMR (CDCl₃, 300 MHz): δ = 7.69-7.59 (m, 2H), 7.55-7.52 (m, 1H), 7.47 (d, *J=* 7.8 Hz, 1H), 7.43-7.31 (m, 4H), 7.29-7.23 (m, 1H), 6.97 (d, *J=* 8.9 Hz, 2H), 3.87 (s, 3H), 2.46 (s, 3H), 2.43 (s, 3H). ¹³C-NMR (CDCl₃, 126 MHz): δ = 158.8 (Cq), 152.9 (Cq), 149.4 (Cq), 138.8 (Cq), 137.7 (Cq), 137.3 (Cq), 131.9 (CH), 131.5 (CH), 131.4 (CH), 131.1 (Cq), 130.4 (CH), 128.7 (CH), 124.0 (CH), 120.0 (CH), 116.0 (CH), 113.0 (CH), 55.3 (CH₃), 21.4 (CH₃), 21.2 (CH₃). IR (neat): 2962, 2914, 2856, 1606, 1518, 1249, 1177, 1016, 816, 791, 689, 538 cm⁻¹. MS (EI) *m*/*z* (relative Intensität): 316 ([M⁺] 100), 301 (40), 197 (67), 182 (65), 153 (42), 91 (78), 65 (30). HR-MS (EI) *m*/*z* berechnet für C₂₁H₂₀N₂O [M⁺] 316.1576, gefunden 316.1577.

### Beispiel 13: (E)-1-(Biphenyl-2-yl)-2-phenyldiazen

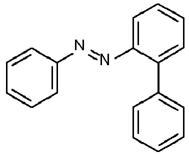

In einem ausgeheizten Reaktionsgefäß wurde eine Suspension bestehend aus (*E*)-1,2-Diphenyldiazen (182 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und Brombenzol (79 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n*-Hexan/EtOAc/NEt₃: 88/6/6). Es wurde (E)-1-(Biphenyl-2-yl)-2-phenyldiazen (68 mg, 53%) als oranges viskoses Öl erhalten.
¹H-NMR (CDCl₃, 300 MHz): δ = 7.81-7.75 (m, 2H), 7.62-7.35 (m, 12H). ¹³C-NMR (CDCl₃, 126 MHz): δ=152.9 (Cq), 149.8 (Cq), 141.2 (Cq), 138.9 (Cq), 131.1 (CH), 131.0 (CH), 130.9 (CH), 130.8 (CH), 129.1 (CH), 128.1 (CH), 127.7 (CH), 127.3 (CH), 123.3 (CH), 116.0 (CH). IR (neat): 3058, 3030, 1470, 1149, 1008, 770, 730, 685, 535, 497 cm⁻¹. MS (EI) *m*/*z* (relative Intensität): 258 ([M⁺] 42), 152 (82), 84 (100), 77 (70). HR-MS (EI) *m*/*z* berechnet für C₁₈H₁₄N₂ [M⁺] 258.1157, gefunden 258.1152.

### Beispiel 14: Methyl-2'-[(E)-phenyldiazenyl]biphenyl-4-carboxylat

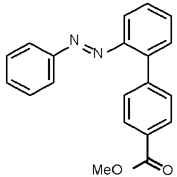

In einem ausgeheizten Reaktionsgefäß wurde eine Suspension bestehend aus (*E*)-1,2-Diphenyldiazen (182 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und Methyl-4-Brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n-*Hexan/CH₂Cl₂: 7/3). Es wurde Methyl-2'-[(E)-phenyldiazenyl]biphenyl-4-carboxylat (93 mg, 59%) als oranger Feststoff erhalten.
Smp. = 128-129 °C. ¹H-NMR (CDCl₃, 500 MHz): δ = 8.09 (d, *J=* 8.6 Hz, 2H), 7.80-7.72 (m, 3H), 7.57-7.52 (m, 4H), 7.51-7.42 (m, 4H), 3.94 (s, 3H). ¹³C-NMR (CDCl₃, 126 MHz): δ=167.2 (Cq), 152.7 (Cq), 149.6 (Cq), 143.7 (Cq), 140.2 (Cq), 131.1 (CH), 130.9 (CH), 130.9 (CH), 130.7 (CH), 129.1 (CH), 128.9 (CH), 128.8 (Cq), 128.7 (CH), 123.3 (CH), 116.0 (CH), 52.1 (CH₃). IR (neat): 3071, 2947, 2920, 2848, 1721, 1437, 1273, 1103, 774, 736, 686, 541 cm⁻¹. MS (EI) *m*/*z* (relative Intensität): 316 ([M⁺] 58), 301 (100), 257 (40), 211 (44), 152 (91), 77 (94). HR-MS (EI) *m*/*z* berechnet für C₂₀H₁₆N₂O₂ [M⁺] 316.1212, gefunden 316.1205.

### Beispiel 15: Methyl-3'-methyl-2'-[(E)-(2-methylphenyl)diazenyl]biphenyl-4-carboxylat

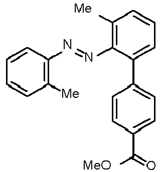

In einem ausgeheizten Reaktionsgefäß wurde eine Suspension bestehend aus (E)-1,2-Di-*o*-tolyldiazen (210 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und Methyl-4-Brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n-*Hexan/CH₂Cl₂: 7/3). Es wurde Methyl-3'-methyl-2'-[(E)-(2-methylphenyl)diazenyl]biphenyl-4-carboxylat (103 mg, 60%) als oranger Feststoff erhalten.
Smp. = 123-124 °C. ¹H-NMR (CDCl₃, 500 MHz): δ = 7.97 (d, *J=* 8.1 Hz, 2H), 7.38-7.26 (m, 9H), 3.92 (s, 3H), 2.47 (s, 3H), 2.28 (s, 3H). ¹³C-NMR (CDCl₃, 126 MHz): δ=167.1 (Cq), 150.9 (Cq), 150.7 (Cq), 145.4 (Cq), 138.5 (Cq), 134.7 (Cq), 131.3 (CH), 131.2 (CH), 131.1 (CH), 130.8 (Cq), 130.1 (CH), 129.1 (CH), 128.9 (CH), 128.1 (CH), 128.0 (Cq), 126.3 (CH), 115.0 (CH), 52.0 (CH₃), 19.2 (CH₃), 17.1 (CH₃). IR (neat): 3059, 2951, 2923, 2844, 1719, 1608, 1398, 1272, 1179, 1101, 856, 766, 739, 712 cm⁻¹. MS (EI) *m*/*z* (relative Intensität): 344 ([M⁺] 60), 329 (93), 285 (30), 225 (50), 165 (99), 91 (100), 65 (34). HR-MS (EI) *m*/*z* berechnet für C₂₂H₂₀N₂O₂ [M⁺] 344.1525, gefunden 344.1526.

### Beispiel 16: Methyl-5'-methyl-2'-[(E)-(4-methylphenyl)diazenyl]biphenyl-4-carboxylat

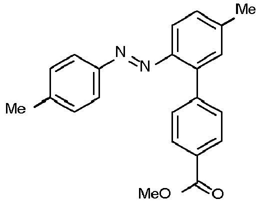

In einem ausgeheizten Reaktionsgefäß wurde eine Suspension bestehend aus (*E*)-1,2-Di *p*-tolyldiazen (210 mg, 1.0 mmol), [{RuC₂(*p*-Cymol)}₂] (15.3 mg, 5.0 mol %), MesCO₂H (24.6 mg, 30 mol %), K₂CO₃ (138 mg, 1.0 mmol) und Methyl-4-Brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n-*Hexan/CH₂Cl₂: 7/3). Es wurde Methyl-5'-methyl-2'-[(E)-(4-methylphenyl)diazenyl]biphenyl-4-carboxylat (112 mg, 65%) als oranger Feststoff erhalten.
Smp. = 138-139 °C. ¹H-NMR (CDCl₃, 500 MHz): δ = 8.08 (d, *J=* 8.6 Hz, 2H), 7.71 (d, *J=* 8.2 Hz, 1H), 7.64 (d, *J=* 8.2 Hz, 2H), 7.54 (d, *J=* 8.6 Hz, 2H), 7.37-7.35 (m, 1H), 7.27 (ddq, *J*= 8.2, 2.0, 0.6 Hz, 1H), 7.25-7.21 (m, 2H), 3.94 (s, 3H), 2.46 (s, 3H), 2.39 (s, 3H). ¹³C-NMR (CDCl₃, 126 MHz): δ=167.2 (Cq), 151.0 (Cq), 147.6 (Cq), 143.9 (Cq), 141.4 (Cq), 141.1 (Cq), 140.0 (Cq), 131.1 (CH), 130.8 (CH), 129.7 (CH), 129.5 (CH), 128.7 (CH), 128.6 (Cq), 123.1 (CH), 115.8 (CH), 52.1 (CH₃), 21.5 (CH₃). IR (neat): 3029, 2948, 2921, 2844, 1721, 1599, 1437, 1274, 1149, 1112, 824, 702, 565, 385 cm⁻¹. MS (EI) *m*/*z* (relative Intensität): 344 ([M⁺] 66), 329 (73), 285 (29), 225 (47), 165 (86), 91 (100), 65 (25). HR-MS (ESI) *m*/*z* berechnet für C₂₂H₂₁N₂O₂ [M+H⁺] 345.1603, gefunden 345.1599.

### Beispiel 17: Methyl-4'-ethyl-2'-[(E)-(3-ethylphenyl)diazenyl]biphenyl-4-carboxylat

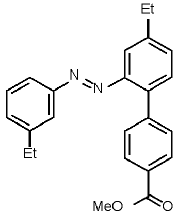

In einem ausgeheizten Reaktionsgefäß wurde eine Suspension bestehend aus (*E*)-1,2-Bis(3-ethylphenyl)diazen (238 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und Methyl-4-Brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n*-Hexan/CH₂Cl₂: 7/3). Es wurde Methyl-4'-ethyl-2'-[(E)-(3-ethylphenyl)-diazenyl]biphenyl-4-carboxylat (155 mg, 83%) als oranger Feststoff erhalten. Smp. = 81-82 °C. ¹H-NMR (CDCl₃, 500 MHz): δ= 8.07 (d, *J=* 8.6 Hz, 2H), 7.68-7.65 (m, 1H), 7.61-7.59 (m, 1H), 7.57-7.52 (m, 3H), 7.50 (dd, *J=* 7.9, 0.5 Hz, 1H), 7.39 (dd, *J=* 7.9, 1.8 Hz, 1H), 7.35 (t, *J=* 7.7 Hz, 1H), 7.29-7.26 (m, 1H), 3.94 (s, 3H), 2.77 (q, *J=* 7.6 Hz, 2H), 2.71 (q, *J*= 7.6 Hz, 2H), 1.32 (t, *J=* 7.6 Hz, 3H), 1.26 (t, *J=* 7.6 Hz, 3H). ¹³C-NMR (CDCl₃, 126 MHz): δ = 167.2 (Cq), 153.0 (Cq), 149.6 (Cq), 145.3 (Cq), 145.2 (Cq), 143.8 (Cq), 137.5 (Cq), 130.9 (CH), 130.7 (CH), 130.6 (CH), 130.5 (CH), 129.0 (CH), 128.8 (CH), 128.6 (Cq), 123.4 (CH), 119.8 (CH), 115.0 (CH), 52.1 (CH₃), 28.7 (CH₂), 28.7 (CH₂), 15.4 (CH₃), 15.3 (CH₃). IR (neat): 2962, 2930, 2871, 1717, 1606, 1439, 1273, 1181, 1102, 691 cm⁻¹. MS (EI) *m*/*z* (relative Intensität): 372 ([M⁺] 89), 357 (100), 313 (45), 239 (61), 180 (35), 165 (75), 105 (91), 77 (32). HR-MS (ESI) *m*/*z* berechnet für C₂₄H₂₅N₂O₂ [M+H⁺] 373.1916, gefunden 373.1915.

### Beispiel 18: Methyl-4'-isopropyl-2'-[(E)-(3-isopropylphenyl)diazenyl]biphenyl-4-carboxylat

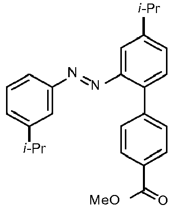

In einem ausgeheizten Reaktionsgefäß wurde eine Suspension bestehend aus (*E*)-1,2-Bis(3-*iso*propylphenyl)diazen (266 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und Methyl-4-Brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n*-Hexan/CH₂Cl₂: 4/6). Es wurde Methyl-4'-isopropyl-2'-[(E)-(3-isopropylphenyl)diazenyl]biphenyl-4-carboxylat (160 mg, 80%) als oranger Feststoff erhalten. Smp. = 92-93 °C. ¹H-NMR (CDCl₃, 500 MHz): δ = 8.07 (d, *J*= 8.5 Hz, 2H), 7.71 (t, *J=* 1.8 Hz, 1H), 7.63 (d, *J=* 1.9 Hz, 1H), 7.56-7.50 (m, 4H), 7.43 (dd, *J=* 8.0, 1.9 Hz, 1H), 7.35 (t, *J*= 7.7 Hz, 1H), 7.32-7.29 (m, 1H), 3.93 (s, 3H), 3.04 (sept, *J*= 6.9 Hz, 1H), 2.97 (sep, *J*= 6.9 Hz, 1H), 1.33 (d, *J=* 6.9 Hz, 6H), 1.27 (d, *J*= 6.9 Hz, 6H). ¹³C-NMR (CDCl₃, 126 MHz): δ = 167.2 (Cq), 153.1 (Cq), 150.0 (Cq), 149.9 (Cq), 149.5 (Cq), 143.8 (Cq), 137.7 (Cq), 130.9 (CH), 130.6 (CH), 129.4 (CH), 129.1 (CH), 129.0 (CH), 128.8 (CH), 128.6 (Cq), 122.4 (CH), 119.6 (CH), 113.7 (CH), 52.1 (CH₃), 34.1 (CH), 34.0 (CH), 23.9 (CH₃), 23.8 (CH₃). IR (neat): 2959, 2889, 2868, 1718, 1607, 1439, 1273, 1113, 858, 835, 797, 694 cm⁻¹. MS (EI) *m*/*z* (relative Intensität): 400 ([M⁺] 96), 385 (100), 341 (41), 253 (45), 211 (47), 179 (43), 119 (78), 91 (42). HR-MS (EI) *m*/*z* berechnet für C₂₆H₂₈N₂O₂ [M⁺] 400.2151, gefunden 400.2138.

### Beispiel 19: Methyl-4'-methoxy-2'-[(E)-(3-methoxyphenyl)diazenyl]biphenyl-4-carboxylat

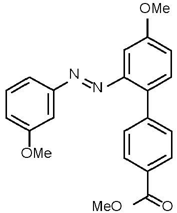

In einem ausgeheizten Reaktionsgefäß wurde eine Suspension bestehend aus (*E*)-1,2-Bis(3-methoxyphenyl)diazen (242 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und Methyl-4-Brombenzoat (108 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n*-Hexan/CH₂Cl₂: 4/6). Es wurde Methyl-4'-methoxy-2'-[(E)-(3-methoxyphenyl)diazenyl]biphenyl-4-carboxylat (139 mg, 74%) als oranger Feststoff erhalten. Smp. = 145-146 °C. ¹H-NMR (CDCl₃, 500 MHz): δ = 8.06 (d, *J=* 8.6 Hz, 2H), 7.53-7.48 (m, 3H), 7.44 (ddd, *J*= 7.8, 1.7, 1.0 Hz, 1H), 7.37 (d, *J*= 8.1 Hz, 1H), 7.35-7.33 (m, 1H), 7.27 (dd, *J=* 2.6, 1.7 Hz, 1H), 7.13 (dd, *J=* 8.5, 2.7 Hz, 1H), 7.02-6.98 (m, 1H), 3.93 (s, 3H), 3.91 (s, 3H), 3.78 (s, 3H). ¹³C-NMR (CDCl₃, 126 MHz): δ=167.1 (Cq), 160.3 (Cq), 160.0 (Cq), 153.9 (Cq), 150.0 (Cq), 143.5 (Cq), 133.5 (Cq), 131.6 (CH), 130.9 (CH), 129.8 (CH), 128.7 (CH), 128.4 (Cq), 118.4 (CH), 118.0 (CH), 117.4 (CH), 106.2 (CH), 99.3 (CH), 55.6 (CH₃), 55.3 (CH₃), 52.1 (CH₃). IR (neat): 2950, 2902, 2834, 1719, 1597, 1519, 1481, 1433, 1270, 1132, 1103, 1039, 887, 782, 683 cm⁻¹. MS (EI) *m*/*z* (relative Intensität): 376 ([M⁺] 64), 361 (100), 317 (53), 241 (38), 182 (35), 139 (54), 107 (65), 77 (38). HR-MS (ESI) *m*/*z* berechnet für C₂₂H₂₁N₂O₄ [M+H⁺] 377.1501, gefunden 377.1491.

### Beispiel 20: (E)-1-(3',4'-Dichlorbiphenyl-2-yl)-2-phenyldiazen

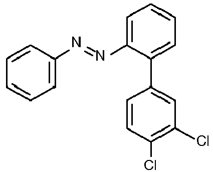

In einem ausgeheizten Reaktionsgefäß wurde eine Suspension bestehend aus (E)-1,2-Diphenyldiazen (182 mg, 1.0 mmol), [{RuCL₂(*p*-Cymol)}₂] (15.3 mg, 5.0 mol %), MesCO₂H (24.6 mg, 30 mol %), K₂CO₃ (138 mg, 1.0 mmol) und 4-Brom-1,2-dichlorbenzol (113 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n-*Hexan/EtOAc/NEt₃: 88/6/6). Es wurde (E)-1-(3',4'-Dichlorbiphenyl-2-yl)-2-phenyldiazen (79 mg, 48%) als oranger Feststoff erhalten.
Smp. = 128-129 °C. ¹H-NMR (CDCl₃, 600 MHz): δ = 7.81-7.77 (m, 3H), 7.62 (d, *J*= 2.1 Hz, 1H), 7.54-7.52 (m, 2H), 7.51-7.45 (m, 5H), 7.29 (dd, *J=* 8.3, 2.1 Hz, 1H). ¹³C-NMR (CDCl₃, 126 MHz): δ=152.8 (Cq), 149.4 (Cq), 139.0 (Cq), 138.9 (Cq), 132.5 (CH), 131.8 (Cq), 131.6 (Cq), 131.3 (CH), 131.1 (CH), 130.5 (CH), 130.3 (CH), 129.5 (CH), 129.2 (CH), 128.9 (CH), 123.3 (CH), 116.1 (CH). IR (neat): 3092, 3055, 1459, 1374, 1137, 1023, 817, 772, 755, 740, 684, 551 cm⁻¹. MS (EI) *m*/*z* (relative Intensität): 326 ([M⁺] 35), 221 (26), 186 (72), 151 (26), 105 (28), 77 (100), 51 (30). HR-MS (ESI) *m*/*z* berechnet für C₁₈H₁₃Cl₂N₂ [M+H⁺] 327.0456, gefunden 327.0451.

### Beispiel 21: (E)-1-(4'-Chlor-4-methylbiphenyl-2-yl)-2-(3-methylphenyl)diazen

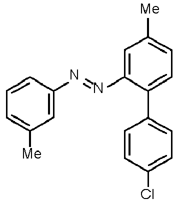

In einem ausgeheizten Reaktionsgefäß wurde eine Suspension bestehend aus (*E*)-1,2-Di-*m-*tolyldiazen (210 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und 1-Brom-4-chlorbenzol (96 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n*-Hexan/EtOAc/NEt₃: 88/6/6). Es wurde (E)-1-(4'-Chlor-4-methylbiphenyl-2-yl)-2-(3-methylphenyl)diazen (93 mg, 58%) als oranger Feststoff erhalten.
Smp. = 120-121 °C. ¹H-NMR (CDCl₃, 500 MHz): δ = 7.73-7.69 (m, 1H), 7.60 (s, 1H), 7.58-7.52 (m, 2H), 7.42 (d, *J=* 7.9 Hz, 1H), 7.38-7.36 (m, 3H), 7.36-7.32 (m, 2H), 7.29-7.25 (m, 1H), 2.45 (s, 3H), 2.41 (s, 3H). ¹³C-NMR (CDCl₃, 126 MHz): δ = 152.9 (Cq), 149.5 (Cq), 139.0 (Cq), 138.5 (Cq), 137.3 (Cq), 137.1 (Cq), 133.2 (Cq), 132.1 (CH), 131.8 (CH), 131.7 (CH), 130.4 (CH), 128.9 (CH), 127.7 (CH), 124.2 (CH), 120.0 (CH), 116.2 (CH), 21.4 (CH₃), 21.2 (CH₃). IR (neat): 3049, 3028, 2949, 2920, 2859, 1596, 1479, 1092, 1005, 811, 788, 747, 687 cm⁻¹. MS (EI) *m*/*z* (relative Intensität): 320 ([M⁺] 67), 201 (54), 166 (93), 91 (100), 65 (35). HR-MS (ESI) *m*/*z* berechnet für C₂₀H₁₈ClN₂ [M+H⁺] 321.1159, gefunden 321.1141.

### Beispiel 22: (E)-1-(3',4'-Dichlor-4-methylbiphenyl-2-yl)-2-(3-methylphenyl)diazen

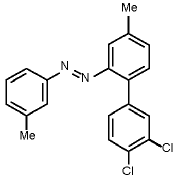

In einem ausgeheizten Reaktionsgefäß wurde eine Suspension bestehend aus (*E*)-1,2- Di-*m-*tolyldiazen (210 mg, 1.0 mmol), [{RuCl₂(*p*-Cymol)}₂] (15.3 mg, 5.0 mol %), MesCO₂H (24.6 mg, 30 mol %), K₂CO₃ (138 mg, 1.0 mmol) und 4-Brom-1,2-dichlorbenzol (113 mg, 0.5 mmol) in trockenem 1,4-Dioxan (2.0 mL) bei 120 °C für 18 h in einer Stickstoffatmosphäre gerührt. Das Reaktionsgemisch wurde dann bei 23 °C mit CH₂Cl₂ (75 mL) verdünnt, über Celite und Kieselgel filtriert und das Filtrat eingeengt. Das so erhaltene Rohprodukt wurde durch Chromatografie an Kieselgel gereinigt (*n*-Hexan/EtOAc/NEt₃: 88/6/6). Es wurde (E)-1-(3',4'-Dichlor-4-methylbiphenyl-2-yl)-2-(3-methylphenyl)diazen (112 mg, 63%) als hell oranger Feststoff erhalten. Smp. = 127-128 °C. ¹H-NMR (CDCl₃, 500 MHz): δ = 7.62-7.56 (m, 4H), 7.46 (d, *J=* 8.3 Hz, 1H), 7.42 (d, *J=* 7.8 Hz, 1H), 7.38-7.33 (m, 2H), 7.29-7.25 (m, 2H), 2.46 (s, 3H), 2.42 (s, 3H). ¹³C-NMR (CDCl₃, 126 MHz): δ = 152.9 (Cq), 149.1 (Cq), 139.1 (Cq), 139.0 (Cq), 138.8 (Cq), 136.0 (Cq), 132.6 (CH), 131.9 (CH), 131.8 (CH), 131.7 (Cq), 131.3 (Cq), 130.2 (CH), 130.1 (CH), 129.4 (CH), 129.0 (CH), 123.7 (CH), 120.5 (CH), 116.2 (CH), 21.4 (CH₃), 21.2 (CH₃). IR (neat): 3026, 2918, 2858, 1601, 1463, 1371, 1133, 1027, 882, 826, 808, 686 cm⁻¹. MS (EI) *m*/*z* (relative Intensität): 354 ([M⁺] 55), 235 (43), 200 (62), 165 (61), 91 (100), 65 (36). HR-MS (EI) *m*/*z* berechnet für C₂₀H₁₆Cl₂N₂ [M⁺] 354.0691, gefunden 354.0686.

Vergleichsbeispiel 1: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass N,N-Dimethylformamid anstelle von 1,4-Dioxan als Lösungsmittel verwendet wurde. Es wurde kein Zielprodukt erhalten.

Vergleichsbeispiel 2: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass N,N-Dimethylacetamid anstelle von 1,4-Dioxan als Lösungsmittel verwendet wurde. Es wurde kein Zielprodukt erhalten.

Vergleichsbeispiel 3: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass Methanol anstelle von 1,4-Dioxan als Lösungsmittel verwendet wurde. Es wurde kein Zielprodukt erhalten.

Vergleichsbeispiel 4: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass N-Methyl-pyrrolidon anstelle von 1,4-Dioxan als Lösungsmittel verwendet wurde. Es wurde kein Zielprodukt erhalten.

Vergleichsbeispiel 5: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass γ-Valerolacton anstelle von 1,4-Dioxan als Lösungsmittel verwendet wurde. Es wurde kein Zielprodukt erhalten.

Vergleichsbeispiel 6: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass Dimethylsulfoxid anstelle von 1,4-Dioxan als Lösungsmittel verwendet wurde. Es wurde kein Zielprodukt erhalten.

Vergleichsbeispiel 7: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass Wasser anstelle von 1,4-Dioxan als Lösungsmittel verwendet wurde. Es wurde kein Zielprodukt erhalten.

Vergleichsbeispiel 8: Methyl-4'-methyl-2'-[(E)-(3-methylphenyl)diazenyl]biphenyl-4-carboxylat Der Versuch wurde wie für Beispiel 1 beschrieben durchgeführt, mit dem Unterschied, dass Essigsäure anstelle von 1,4-Dioxan als Lösungsmittel verwendet wurde. Es wurde kein Zielprodukt erhalten.

### Beispiel 23: Methyl-2'-amino-4'-methylbiphenyl-4-carboxylat

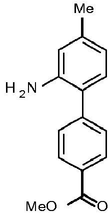

In einem ausgeheizten Reaktionsgefäß wurde eine Suspension bestehend aus (*E*)-1,2-Di-*m-*tolyldiazen (421 mg, 2.0 mmol), [{RuCl₂(*p*-Cymol)}₂] (30.6 mg, 5.0 mol %), MesCO₂H (49.3 mg, 30mol%), K₂CO₃ (276 mg, 2.0 mmol) und Methyl-4-brombenzoat (215 mg, 1.0 mmol) in trockenem 1,4-Dioxan (3.0 mL) bei 120 °C für 18 h in einer Stickstoffatmosphäre gerührt. Dem Reaktionsgemisch wurde dann bei 23 °C [RuCl₂(PPh₃)₃] (47.9 mg, 5.0 mol %), KOH (16.8 mg, 30 mol %), Zn (262 mg, 4.0 mmol), H₂O (14.4 mg, 8.0 mmol) und abschließend 1,4-Dioxan (2.0 mL) zugegeben. Es wurde bei 80 °C für 24 h in Stickstoffatmosphäre gerührt. Das Reaktionsgemisch wurde dann bei 23 °C mit EtOAc (75 mL) verdünnt, über Celite und Kieselgel filtriert und das Filtrat eingeengt. Das so erhaltene Rohprodukt wurde durch Chromatografie an Kieselgel gereinigt (*n-*Hexan/EtOAc: 5/1). Es wurde Methyl-2'-amino-4'-methylbiphenyl-4-carboxylat (160 mg, 66%) als weißer Feststoff erhalten.
Smp. = 136-137 °C. ¹H-NMR (CDCl₃, 300 MHz): δ = 8.08 (d, *J=* 8.6 Hz, 2H), 7.52 (d, *J=* 8.6 Hz, 2H), 7.02 (d, *J=* 7.7 Hz, 1H), 6.65 (ddd, *J*= 7.7, 1.6, 0.7 Hz, 1H), 6.59 (s, 1H), 3.92 (s, 3H), 3.71 (s, 2H), 2.30 (s, 3H). ¹³C-NMR (CDCl₃, 400 MHz): δ = 166.9 (Cq), 144.5 (Cq), 143.2 (Cq), 139.2 (Cq), 130.2 (CH), 130.0 (CH), 129.0 (CH), 128.6 (Cq), 123.7 (Cq), 119.8 (CH), 116.5 (CH), 52.1 (CH₃), 21.2 (CH₃). IR (neat): 3442, 3360, 2947, 2915, 2164, 1703, 1604, 1435, 1280, 1178, 1103, 772 cm⁻¹. MS (EI) *m*/*z* (relative Intensität): 241 ([M⁺] 100), 210 (31), 167 (35), 84 (24), 49 (38). HR-MS (EI) *m*/*z* berechnet für C₁₅H₁₅NO₂ [M⁺] 241.1103, gefunden 241.1109.

## Patentansprüche

1. Verfahren zur Herstellung von Biphenylaminen der allgemeinen Formel (I) 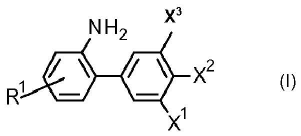 in welcher
R¹ für Wasserstoff, Hydroxy, Fluor, Chlor, C₁-C₄-Alkyl, C₁-C₄-Alkoxy, C₁-C₄-Alkylthio oder C₁-C₄-Halogenalkyl steht,
X¹ für Wasserstoff" Alkoxy, Alkanoyl, Alkylcarboxylat, Fluor oder Chlor steht,
X² für Wasserstoff, Alkoxy, Alkanoyl, Alkylcarboxylat, Fluor oder Chlor steht.,
X³ für Wasserstoff, Alkoxy, Alkanoyl, Alkylcarboxylat, Fluor oder Chlor steht.
**dadurch gekennzeichnet, daß** man in einem ersten Schritt (1) Azobenzole der Formel (II) 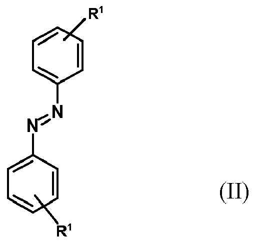 in welcher
R¹ die oben angegebene Bedeutung hat,
mit einer aromatischen Verbindung der Formel (III) 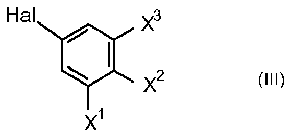 in welcher
X¹, X² und X³ die oben angegebenen Bedeutungen haben, und
Hal für Iod, Brom oder Chlor
steht,
in Gegenwart eines Katalysatorsystems bestehend aus einem Rutheniumkatalysator, einem Aktivierungsmittel und einer Base, umsetzt und
in einem zweiten Schritt (2) die so erhaltenen Azobenzole der Formel (IV) 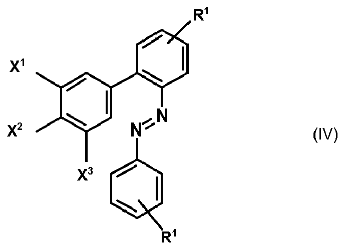 in welcher R¹, X¹, X² und X³ die oben angegebenen Bedeutungen haben,
zu den Biphenylaminen der Formel (I) hydriert.

2. Verfahren nach Anspruch 1, **dadurch gekennzeichnet, daß** das Lösungsmittel ausgewählt ist aus der Gruppe, die Ketone, Nitrile, Ether, Kohlenwasserstoffe und halogenierte Kohlenwasserstoffe und Alkohole sowie Mischungen dieser Lösungmittel umfaßt.

3. Verfahren nach Anspruch 1 oder 2, **dadurch gekennzeichnet, daß** das Lösungsmittel ausgewählt ist aus der Gruppe, die 1,4-Dioxan, Toluol, ortho-Xylol, meta-Xylol, para-Xylol sowie Mischungen dieser Lösungmittel umfaßt.

4. Verfahren nach einem der vorhergehenden Ansprüche, **dadurch gekennzeichnet, daß** der Katalysator [{RuCl₂(p-Cymol)}₂] ist.

5. Verfahren nach einem der vorhergehenden Ansprüche, **dadurch gekennzeichnet, daß** das Aktivierungsmittel eine Säure, vorzugsweise eine Carbonsäure, ist.

6. Verfahren nach einem der vorhergehenden Ansprüche, **dadurch gekennzeichnet, daß** das Aktivierungsmittel eine Carbonsäure ausgewählt aus der Gruppe ist, die besteht aus: Ameisensäure, Essigsäure, Propionsäure, Pivalsäure, Benzoesäure, 2-Methylbenzoesäure, 3-Methylbenzoesäure, 4-Methylbenzoesäure, 2,3-Dimethylbenzoesäure, 2,4-Dimethylbenzoesäure, 2,5-Dimethylbenzoesäure, 2,6-Dimethylbenzoesäure, 3,4-Dimethylbenzoesäure, 3,5-Dimethylbenzoesäure, 2,4,6-Trimethylbenzoesäure, 2,3,4-Trimethylbenzoesäure, 3,4,5-Trimethylbenzoesäure, 2,3,5-Trimethylbenzoesäure, 2,3,6-Trimethylbenzoesäure, Phenylessigsäure, 2-Methylphenylessigsäure. 3-Methylphenylessigsäure, 4-Methylphenylessigsäure, 2,5-Dimethylphenylessigsäure, 2,3,6-Trimethylphenylessigsäure, 2,3,5,6-Tetramethylphenylessigsäure, 2,3,4,6-Tetramethylphenylessigsäure, 2-Chlorphenylessigsäure, 3-Chlorphenylessigsäure, 4-Chlorphenylessigsäure, 2,4-Dichlorphenylessigsäure.

7. Verfahren nach einem der vorhergehenden Ansprüche, **dadurch gekennzeichnet, daß** das Aktivierungsmittel 2,4,6-Trimethylbenzoesäure ist.

8. Verfahren nach einem der vorhergehenden Ansprüche, **dadurch gekennzeichnet, daß** die Base eine anorganische Base ist.

9. Verfahren nach einem der vorhergehenden Ansprüche, **dadurch gekennzeichnet, daß** die Base ausgewählt aus der Gruppe bestehend aus: Lithiumhydroxid, Natriumhydroxid, Kaliumhydroxid, Lithiumcarbonat, Natriumcarbonat, Kaliumcarbonat, Lithiumhydrogencarbonat, Natriumhydrogencarbonat, Kaliumhydrogencarbonat, Calciumcarbonat, Magnesiumcarbonat.

10. Verfahren nach einem der vorhergehenden Ansprüche, **dadurch gekennzeichnet, daß** die Base Kaliumcarbonat ist.

11. Verfahren nach einem der vorhergehenden Ansprüche, **dadurch gekennzeichnet, daß** das Aktivierungsmittel in Mengen von 0,1 bis 100 Molprozent bezogen auf die aromatische Verbindung der Formel (III) eingesetzt wird.

12. Verfahren nach einem der vorhergehenden Ansprüche, **dadurch gekennzeichnet, daß** der Rutheniumkatalysator in Mengen von 1 bis 20 Molprozent bezogen auf die aromatische Verbindung der Formel (III) eingesetzt wird.

13. Verfahren nach einem der vorhergehenden Ansprüche, **dadurch gekennzeichnet, daß** sas Molverhältnis von Azobenzol der Formel (II) zu Halogenaromat der Formel (III) 1 zu 0,4 bis 1 ist.

14. Verfahren nach einem der vorhergehenden Ansprüche, **dadurch gekennzeichnet, daß** das Molverhältnis von Azobenzol der Formel (II) zu Halogenaromat der Formel (III) 1 zu 0,45 bis 0,9 ist.

15. Verfahren nach einem der vorhergehenden Ansprüche, **dadurch gekennzeichnet, daß** das Lösungsmittel 1,4-Dioxan, der Aktivator 2,4,6-Trimethlylbenzoesäure , die aromatische Verbindung der Formel (III) eine bromierte aromatische Verbindung, die Base Kaliumcarbonat und der Katalysator [{RuCl₂(p-Cymol)}₂] ist.
